(19) Europäisches Patentamt

European Patent Office

Office européen des brevets

(11) **EP 0 885 954 A1**

(12) **EUROPEAN PATENT APPLICATION**

(43) Date of publication:
23.12.1998 Bulletin 1998/52

(21) Application number: 97201833.7

(22) Date of filing: 16.06.1997

(51) Int. Cl.$^6$: **C12N 9/24**, C12N 1/14, C12N 11/12, C12P 19/26, C12P 21/02, C12Q 1/34, A23L 3/3571 // (C12N1/14, C12R1:80)

(84) Designated Contracting States:
AT BE CH DE DK ES FI FR GB GR IE IT LI LU MC NL PT SE
Designated Extension States:
AL LT LV RO SI

(71) Applicant:
SOCIETE DES PRODUITS NESTLE S.A.
1800 Vevey (CH)

(72) Inventors:
- **Raetz, Eric**
  **1012 Lausanne (CH)**
- **Leuba, Jean-Louis**
  **1034 Boussens (CH)**
- **Di Giambattista, Roberta**
  **01100 Viterbo (IT)**
- **Federici, Federico**
  **06100 Perugia (IT)**
- **Fenice, Massimiliano**
  **01100 Viterbo (IT)**

(54) **Chitinolytic enzymes production by Penicillium janthinellum**

(57) The invention relates to (1) chitinolytic enzymes produced by *P. janthinellum* cells, having a molecular mass by SDS-PAGE of about 40000-60000, (2) a process for producing chitinolytic enzymes or N-acetyl-glucosamine, in which *P. janthinellum* cells are maintained in a medium under conditions suitable for the production of chitinolytic enzymes. Suitably, *P. janthinellum* are immobilised on a modified macroporous cellulose carrier and chitinolytic enzymes N-acetyl-glucosamine are continuously produced. These chitinolytic enzymes are particularly adapted for the production of chitin degradation products and for inhibiting food spoiling micro-organisms.

Figure 4

## Description

The present invention relates to the production of chitinolytic enzymes by *P. janthinellum,* the use of *P. janthinellum* to hydrolyse chitin containing materials, and the use of chitinolytic enzymes from *P. Janthinellum* to produce chitin degradation products and to preserve foods.

State of the art

Chitin is an insoluble β-1,4 linked polymer of N-acetylglucosamine. It is the most abundant amino-polysaccharide in nature and renewable resource after cellulose. Chitin is widely spread in the living world, constituting the main component of insect, crustacean, nematode shells and fungal cell wall. These chitin-containing organisms produce chitinolytic enzymes above all implicated in moulting or morphogenesis. For example, fungal chitinolytic enzymes could be involved in growth regulation or required for cell separation (Kuranda M.J. et al., J. of Biol. Chem., 266, 19758-19767, 1991). Chitinolytic enzymes could also be involved in the penetration of a host by mycoparasites (Balasubramanian R. et al., Mycologia, 78, 157-163, 1986) or by entomopathogenic fungi (El-Sayed G.M. et al., J. Invertebrates Pathology, 54, 394-403, 1989). Some other organisms which do not contain chitin also produce chitinolytic enzymes. This is the case of plants in which the chitinolytic enzymes production is elicited by pathogens after abiotic stresses. (Sahai A.S. et al., FEMS Microbiology Reviews 4, 317-338, 1993).

It is possible to distinguish several chitinolytic enzymes usually working in combination to hydrolyse chitin: exo- and endo-chitinase (chitin glucanohydrolase), chitobiase and N-acetylglucosaminidase (chitobiose acetylaminoglucohydrolase). Exo-chitinase is defined as an enzyme releasing chitobiose, endo-chitinase as an enzyme splitting within the chitin polymer, and β-N-acetylglucosaminidase an enzymatic form producing N-acetylglucosamine monomers from chitin (Flach J. et al., Experientia, 48, 701-716, 1992). At last, chitobiase hydrolyses chitobiose.

To make a comparison between the chitinolytic activity of different strains on the basis of the literature available is not an easy task. Different authors indeed use different methods, different definitions of enzymatic activity units, different culture conditions and media. Furthermore, constitutive chitinolytic activities are analysed instead of induced ones, and the conversion of the enzymes activity expressed in different ways is difficult and scarcely precise. Table 1 below shows the results of a comparison of different chitinolytic activities reported by literature.

To make a comparison between the chitinolytic activity of different strains on the basis of the literature available is not an easy task. Different authors indeed use different methods, different definitions of enzymatic activity units, different culture conditions and media. Furthermore, constitutive chitinolytic activities are analysed instead of induced ones, and the conversion of the enzymes activity expressed in different ways is difficult and scarcely precise. Table 1 below shows the results of a comparison of different chitinolytic activities reported by literature.

Table 1

| Microoganism | Enzyme activity[a] | | | | |
|---|---|---|---|---|---|
| | Strain | $(\mu/ml)^b$ | $(mU/ml)^b$ | $(\mu/ml)^c$ | $(mU/ml^c$ |
| Aspergillus niger (1) | 120.48 | --- | 100 | --- | --- |
| Aspergillus fumigatus (2) | QM 45h | 11.0 | 92 | 2.6 | 22 |
| Myrothecium verrucaria (2) | QM 460 | 7.0 | 58 | 0.8 | 7 |
| Mucor subtilissimum (2) | QM 1063 | 4.8 | 40 | 1.8 | 15 |
| Conidiobolus villosus (2) | QM 929 | 2.8 | 23 | 0.6 | 5 |
| Penicillium lilacinum (2) | QM 4e | 2.4 | 20 | 0.2 | 2 |
| Trichoderma viride (2) | QM 6a | 1.5 | 13 | 0.4 | 3 |
| Aphanocladium album (3) | ETH 483 | --- | 50 | --- | 50 |
| Phomas sp. (4) | A24 | --- | 167 | --- | --- |
| Penicillium oxalicum (5) | 1670 | --- | 110 | --- | --- |
| Aspergillus carneus (6) | --- | 47.3 | 394 | --- | --- |
| Verticillium lecanii (4) | A3 | --- | 289 | --- | 307 |

(1) Lahoz et al., Mycopathologia, 60, 45-49, 1976.
(2) Monreal et al., Canadian Journal of Microbiology, 15, 689-696, 1968.
(3) Studer et al., FEMS Microb. Lett., 99, 213-216, 1992.
(4) Fenice et al., In: Muzzarelli RAA (Ed.) Chitin Enzimology. European Chitin Society, Lyon and Ancona, 303-310, 1996.
(5) Rodriguez et al., Letters in Applied Microbiology, 16, 69-71, 1993.
(6) Sherief et al., Applied Microbiology and Biotechnology, 35, 228-230, 1991.
[a] Activities are expressed as arbitrary ($\mu$) and, if necessary, after conversion as international units (1 Unit represents the amount of enzyme which releases 1 $\mu$mol of N-acetylglucosamine per ml per min)
[b] Activities on colloidal chitin
[c] Activities on non-purified chitin (e.g. crab shells)

Since chitinolytic enzymes may play a role in food preservation (see J05095784) and may be involved in production of valuable polysaccharides. The industry has also been interested by industrial production of chitinolytic enzymes. For instance, production of chitinolytic enzymes by Micromonospora chalcae was investigated in submerged culture in medium containing 2% chitin, and after co-immobilization with Ca-alginate (O'Riordan et al., Biotechnol. Lett., U, 735-738, 1989). Likewise, chitinolytic enzymes were also produced by double-layered gel fiber-immobilized Wasabia japonica cells in Erlenmeyer flasks (Tanaka et al., J. Ferment. Bioeng., 81, 220-225, 1996).

Production of chito-oligosaccharides and N-acetylglucosamine, the basic unit component of chitin, is very important for an efficient use of chitin since N-acetylglucosamine is useful for producing some chemical and pharmaceutical materials or to complement certain food products. Reports on the possibility of producing N-acetylglucosamine via chitinolytic enzymes on an industrial scale are rather scarce (Carroad P.A. et al., J. of Food Science, 43, 1158-1161, 1978; Cosio I.G. et al., J. of Food Science, 47, 901-905, 1982). This can be explained by the slowness of enzyme reactions on chitin and by the low final yield, thus limiting the economical interest for this process.

However, the production could be improved by fermentation processes using immobilised microbial cell systems where semi-continuous and continuous processes could be possible. So far, no production of chitinolytic enzymes and chitin degradation products, e.g. N-acetylglucosamine, by immobilised fungal cells appears to be reported. Since traditional natural materials for cell immobilisation (e.g. alginate, carrageenan, agar, glass beads, etc.) very often present technical problems (low mechanical stability, low oxygen permeation rate, etc.: Muscat et al., In Immobilised Cells:

basics and application, Wijhels et al. Ed., p 55-61, 1996), the selection of a suitable carrier is also important in order to obtain the high performances required for an immobilised system (Federici, World J. Microbiol. Technol., 9, 495-502, 1996).

## Summary of the Invention

The present invention has for object chitinolytic enzymes, obtainable from *Penicillium janthinellum* cells, having a molecular mass determined by SDS-PAGE of about 40000-60000.

The invention is also concerned with the use of chitinolytic enzymes extracted from *P. janthinellum* cells, for the production of chitin degradation products and for inhibiting food spoiling microorganisms.

The present invention has also for object a process for producing chitinolytic enzymes, in which *P. janthinellum* cells are maintained in a medium under conditions suitable for the production of chitinolytic enzymes.

Finally, the invention has for object the use of *P. janthinellum* cells for the hydrolysis of chitin containing materials.

## Detailed description of the invention

In the context of the present invention, "chitinolytic enzymes" should be understood as all the enzymes having a chitinolytic activity, such as exo- and endo-chitinases, chitobiase and N-acetylglucosaminidase.

The term "a fixed bed of immobilised *P. janthinellum* cells" is understood as cells immobilised on or in a carrier which is compacted in a reactor adapted for the continuous production of chitinolytic enzymes and/or chitin degradation products, e.g. N-acetyl-glucosamine.

The term "a fluidized bed of immobilised *P. janthinellum* cells" is understood as cells immobilised on a carrier, which is suspended but not compacted in a reactor adapted for the continuous production of chitinolytic enzymes and/or chitin degradation products. The flow of medium, circulating from the bottom to the top, then places the carrier particles in suspension. This type of reactor known as a "fluidized bed reactor" is particularly advantageous for the processing of media comprising solid matter in suspension.

The present invention is concerned with any chitinolytic enzymes having structures characterised by the fact that they originate from *P. janthinellum* cells, and having molecular mass by SDS-PAGE of about 40000-60000, preferably about 45000. These enzymes can be isolated from a supernatant culture of *P. janthinellum* by anion exchange chromatography and/or by gel filtration, for instance. Several enzymes can thus be separated, especially exo- and endo-chitinases, chitobiase and N-acetylglucosaminidase.

Most preferably, chitinolytic enzymes from the *P. janthinellum* strain P9 are isolated. This strain was deposited, the 29th April, 1997, at the Collection Nationale de Cultures de Microorganismes (CNCM), 25 rue du Docteur Roux, Paris, France, where it received the deposit number CNCM I-1869. This strain shows a pale grey to greenish-grey colony spreading reaching 5-7 cm diameter after 10 days at 24°C on the standart CzA/Mea medium. The conodiphores present few diverged branches, and are smooth walled or slightly roughened. The conidia are strongly ellipsoidal sometimes later becoming subglobose often with apiculate ends, more or less rough-walled (mostly 3-5 $\mu$m long).

This strain presents a very high potential for production of chitinolytic enzymes when compared to species known to produce chitinolytic enzymes, such as *Verticillium lecanii, Trichoderma species, Aspergillus species, Myrothecium verrucaria, Mucor subtilissimum, Conidiobolus villosus, Penicillium* species and *Aphanocladium album*, for instance.

The invention is also concerned with a process for producing chitinolytic enzymes and/or chitin degradation products, e.g.. N-acetylglucosamine, in which *P. janthinellum* cells are maintained in a medium under conditions suitable for the production of chitinolytic enzymes and chitin degradation products. Any media adapted for the growth or the survival of *P. janthinellum* may be used, suitably a 1% YNB medium containing 1% glucose, for instance. This medium preferably contains, in case production of chitin degradation products is required, chitinaceous materials, such as chitin isolated from crab shell, shrimp shell and fungal cell walls, for instance from *F. oxysporum* and/or *S. rolfii.* Suitably, the chitin material is under colloidal form.

Maintenance of the cells may be effected by way of *P. janthinellum* immobilisation. A surprising advantage of the present invention lies in the fact that it is possible to produce high amounts of chitinolytic enzymes and/or chitin degradation products, e.g. N-acetylglucosamine, by immobilisation of these cells. Any kind of carrier capable of retaining viable *P. janthinellum* cells may be used. Most preferably, the carrier is a modified macroporous cellulose such as the Microcube range of products from Biomaterial Co. (Mitsubishi Corp., Japan), especially the products designated as BR-L01T(500), BR-LO1T(70), FN-SO1T(500), FM-SO1B(70), BZ-HO1T(500), BZ-HO1B(70), CM-LO1T(500) and CM-HO1B(70), for instance.

Finally, chitinolytic enzymes and/or chitin degradation products, e.g.. N-acetylglucosamine, may be isolated by any means known to the skilled person, such as anion exchange chromatography and/or gel filtration for instance.

In a first preferred embodiment of the present invention, chitinolytic enzymes and/or chitin degradation products, e.g.N-acetyl-glucosamine, may be produced in a reactor of the stirred tank type comprising immobilised cells. The system may be automated and operated in a semi-continuous manner with the following three stages. In the first instance, the tank is filled with the medium to be processed, then left to be used by the immobilised cells with mechanical stirring, after which the tank is emptied after a predetermined period. The retention of the cells in the reactor may take place by means of a filter disposed in the lower portion of the tank.

In a second preferred embodiment of the present invention, chitinolytic enzymes and/or chitin degradation products, e.g. N-acetyl-glucosamine, are produced in a reactor comprising a fluidized bed of immobilised cells. When used in this way, the cells retain particularly good survival and produce high amounts of chitinolytic enzymes and/or chitin degradation products.

In a third preferred embodiment of the present invention, instead of using live immobilised cells for producing chitin degradation products, e.g N-acetylglucosamine, chitinolytic enzymes produced by *P. janthinellum* may also be directly used to produce chitin degradation products, by way of immobilization by covalent or non-covalent bonding on a carrier, or by polymerisation of the enzymes adsorbed on a carrier. Methods of immobilisation and selection of suitable carriers are in fact well known to one skilled in the art (see for example EP676145 and EP777972).

In addition, chitinolytic enzymes thus produced may then be used for inhibiting food spoiling micro-organisms and, in particular, micro-organisms selected from *Penicillum carneum, Penicillum granulatum, Penicillum citrinum, Penicillum corylophilum, Penicillum chrysogenum, Penicillum roquefortii Penicillum hirsitum, Penicillum camembertii, Penicillum canescens, Penicillum brevicompactum, Fusarium moniliforme, Fusariumavenaceum, Fusarium equiseti, Fusarium solanii, Cladosporium cladosporoides, Cladosporium herbarum, Paecilomyces vaiotti, Eremicella nidulans, Mucor plumbeus* and *Byssochlamys nivea,* for example.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the claims. Various publications are cited herein, the disclosures of which are incorporated by reference in their entireties to the extent necessary to understand the present invention. In the following description, the percentages are given by weight unless where otherwise stated. These examples are preceded by a brief presentation of the drawings.

Fig. 1 shows the production of chitinolytic activity during fermentation at 25°C by *Penicillum janthinellum* P9 (♦), *Verticillium lecanii* $A_3$ (■), *Trichoderma harzianum* $P_1$ (▲) and *Trichoderma harzianum* $T_{22}$ (X).

Fig. 2 shows the production of chitinolytic activity during fermentation at 5°C by *Penicillum janthinellum* P9 (♦), *Verticillium lecanii* $A_3$ (■), *Trichoderma harzianum* $P_1$ (▲) and *Trichoderma harzianum* $T_{22}$ (X).

Fig. 3 shows the production of chitinolytic activity by free (♦) and immobilized (□) cells of *P. janthinellum* P9 cultivated in repeated-batch processes.

Fig. 4 shows the time course of the continuous chitinolytic enzyme activity production by immobilised cells of *P. janthinellum* P9: ■ = chitinolytic activity; ♦ = mycelium released; ▲ = chitin in the cultural broth.

Example 1 Chitinolytic enzymes production

Four chitinolytic strains (*Penicillium janthinellum* P9, *Verticillium lecanii* A3, *Trichoderma harzianum* P1 and *Trichoderma harzianum* T22) were compared in terms of chitinolytic enzymes production. Experimental conditions were carried out with a medium already selected for different strains (see above Fenice et al, 1996) using a pH (5.5) generally employed for the fungal chitinolytic enzyme production, two temperatures (25 and 5 °C) and a culture shaken at 200 rpm. Even not optimised for all the strains, these conditions could reasonably fit the strains' requirements. Chitinolytic activity was obtained according to the analytical methods described in example 2, after filtration and precipitation in 80% ammonium sulphate.

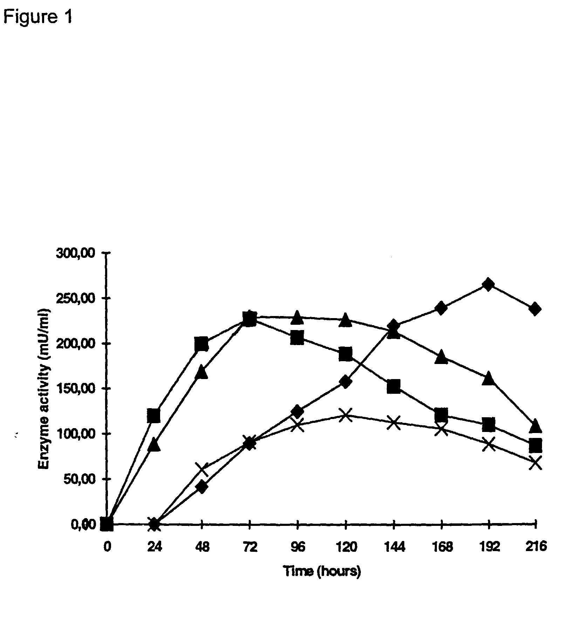

Results presented in figure 1 show the time span of chitinolytic activity released by four chitinolytic strains (*Penicillium janthinellum* P9, *Verticillium lecanii* A3, *Trichoderma harzianum* P1 and *Trichoderma harzianum* T22) in a culture medium containing colloidal chitin as the sole carbon source and incubated at 25° C for 216 h. The highest level of chitinolytic activity (266 mU/ml) was released by *P. janthinellum* P9 after 192 h of incubation. The strains *V. lecanii* and *T. harzianum* P1 released a lower level of chitinolytic activity (ca 230 mU/ml) but their maximum activity was reached after 72 h of incubation.

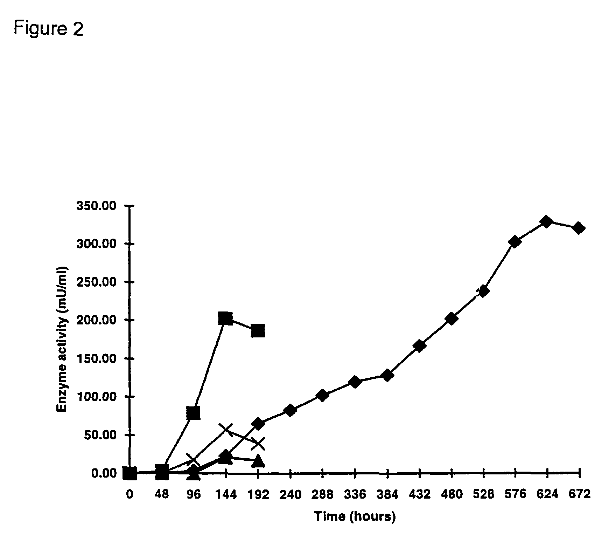

Results presented in figure 2 show the time span of chitinolytic activity for the same strains at 5°C. Here the strain *V. lecanii,* even with a delay of 72 h probably due to the slower growth at low temperature, released its highest level of enzyme activity (ca 200 mU/ml) after 144 h of incubation. The *Trichoderma* strains show a marked reduction in activity with a maximum after 144 h of incubation. *P. janthinellum* P9 shows a very slow increase of the chitinolytic activity; in fact, after 216 h of incubation, its chitinolytic enzymes were 90 mU/ml only. However, the maximal chitinolytic activity reached after 624 h was quite significantly higher for *P. janthinellum* than for other production reference strains.

Example 2 Chitinolytic enzymes purification and characterisation

*P. janthinellum* supernatant of example 1 containing chitinolytic enzymes was filtered, concentrated by membrane ultrafiltration, washed extensively for desalting and, finally, lyophilised.

The lyophilised crude enzyme solution, after resuspension in water and filtration, using 0.22 Millex-GV (Millipore, USA) units, was directly applied to an HQ/M (Poros, Perspective Biosystems Inc, US) anion exchange perfusion chromatography connected to a Biocad workstation, equilibrated with a 20 mM tris-bis-tris propane buffer, pH 7.0, and eluted with a continuous linear gradient of the same buffer containing 0-0.6 M NaCl at a flow rate of 4 ml/min. Fractions of 1 ml were collected. Results show that the main enzyme activities (endo- exo-chitinases and chitobiase) were detected in fractions 28-32 while a minor endo-chitinase activity was detected in fractions 48-53. Fractions containing chitinolytic activities (28-32 and 48-53) were pooled and concentrated by ultrafiltration with Centricon 10 (Amicon, USA) disposable units.

The concentrated samples from ion exchange were loaded separately on a Sephacryl S-200 (Pharmacia, Sweden) column (2.6 X 50 cm) connected with a Biocad workstation, equilibrated with a 20 mM tri-bis-tris propane buffer, pH 7.0, and eluted with the same buffer at a flow rate of 4 ml/min. Molecular markers were bovine albumin (67.000), egg albumin (43.000), chymotrypsinogen A (25.000) and ribonuclease (13.700). The main enzyme activities (endo-chitinase and chitobiase) were detected in fractions 13-19 (originated from fraction 28-32 of ion exchange) while a minor endo-chitinase activity was detected in fractions 8-11 (originated from fractions 48-53 or ion exchange). Fractions containing the enzyme activities were pooled and concentrated by ultrafiltration as already described. Results show that the molecular mass of the fractions containing the chitinolytic activities appears to be around 160.000.

Sodium dodecyl sulphate polyacrylamide gel electrophoresis (SDS-PAGE) was used to check protein purity and to determine the molecular mass of the purified enzymes under denaturing conditions, using a 12% acrylamide gel (Laemmli, 1970). After electrophoresis the gels were stained for 30 min in a solution of 0.2% of Blue-R dye in 30% acetic acid and 10% methanol; then destained in the same solution, without the dye. Molecular markers were those supplied by Biorad (USA) for the high and low standard. Results show that the main enzyme activities (exo-, endo-chitinase and chitobiase, present in fractions 28-32 from ion exchange and 13-19 from gel filtration) have a molecular mass of about 45.000. The minor activity contained in fraction 48-53 from ion exchange and 8-11 / 16-19 from gel filtration presents a very small band at the same molecular weight. It can be noted that the molecular mass of the purified chitinolytic enzymes, obtained by SDS-PAGE, is fairly different from that obtained by gel filtration.

Finaly, isoelectric focusing of the purified enzymes showed that the main chitinolytic activity has an isoelectric point of about pH 4.8.

Example 3 *P. janthinellum immobilisation*

This example reports the production of chitinolytic enzymes by the potent strain *Penicillium janthinellum* P9, immobilised on different carriers (macroporous cellulose with different chemical modifications and polyurethane sponge) and cultivated either in repeated-batch processes or in continuous culture. Different materials and methods were used and are described as follows.

1. Chitin preparation: colloidal chitin was prepared by treating chitin from crab shells (Sigma) with 50% sulphuric acid and resuspending the dissolved material in distilled water. After swelling, the chitin was resuspended in water and sterilized by autoclaving 20 min at 121°C. The solid content of the sterile chitin suspension was evaluated by dry weight determination.

2. Immobilisation medium: the medium used for the cell immobilisation contains $(NH_4)_2SO_4$, 0.25%; $KH_2PO_4$, 0.1%; $MgSO_4.7H_2O$, 0.04%. PH5.0 is achieved without adjustment. After sterilization (121°C for 20 min), 2% filter-sterilized (Millipore membrane HA, 0.45$\mu$m, 47 mm) glucose is added.

3. Production media: the producing media used contains $KH_2PO_4$, 1.0%; $MgSO_4.7H_2O$, 0.04%, supplemented with 1.0% and 0.3% of colloidal chitin. pH is adjusted to 5.0 with 0.5 M $Na_2HPO_4.2H_2O$. The media are autoclaved at 121°C for 20 min.

4. Immobilisation procedure: The following carriers (see table 2 below) of chemically modified macroporous cellulose (Mierocube, Mitsubishi Ltd. Tokyo, Japan) were used: BR-L01T(500); BR-LO1T(70); FN-SO1T(500); FN-SO1B(70); BZ-HO1T(500); BZ-HO1B(70); CM-LO1T(500); CM-HO1B(70). The polyurethane sponge (PUS) cubes used were of ca. 0.3 $cm^3$. Inocula were prepared as follows: 100 ml of immobilization medium plus 20% (v/v) of carrier in a 250 ml Erlenmeyer-flask (E-flask) were sterilized at 121°C for 20 min; to these 2 x $10^6$ conidiospores were inoculated and then incubated in a rotary shaker at 120 rpm and 25°C for 5-6 days.

Table 2

| Carrier | Chemical modification | Electric charge | Shape | Pore size ($\mu$m) |
|---|---|---|---|---|
| BR-L01T(500) | polyethyleneimine | low | cubic | 500 |
| BR-LO1T(70) | polyethyleneimine | low | cubic | 70 |
| FN-SO1T(500) | non electric charged | non modified | cubic | 500 |
| FN-SO1B(70) | non electric charged | non modified | cubic | 70 |
| BZ-HO1T(500) | polyethyleneimine | high | cubic | 500 |
| BZ-HO1B(70) | polyethyleneimine | high | bead | 70 |
| CM-LO1T(500) | carboxymethyl group | low | cubic | 500 |
| CM-LO1T(500) | carboxymethyl group | low | cubic | 70 |

5. Culture conditions: to remove any residual carbon source, the colonised beads were washed in sterile deionised water and then transferred into another flask containing 50 ml of fermentation medium. Cultures were carried out on a rotary shaker at 180 rpm at 28°C. Repeated batch cultivations of the immobilised *P. janthinellum* P9 were carried out removing the exhausted medium from E-flasks and adding 50 ml of fresh production medium every 48 h of incubation. Continuous enzymes production was obtained, as described by Gallo Federici et al. (Appl. Microbiol. Biotechnol., 33, 407-409, 1990) in a 1000 ml (working volume) bubble-column bioreactor filled with production medium and inoculated with 5% (V/V) of carrier colonised as described above. For the induction of chitinolytic activity, the immobilised cells were first incubated as a batch culture (120 h); then fresh medium was continuously fed into the bioreactor at a flow rate of 36 ml/h, unless otherwise indicated. The aeration rate was 1 V/Vm and the temperature 28°C.

6. Estimation of chitin and N-acetylglucosamine: after centrifugation and washing, residual chitin in the effluent was estimated by dry weight determinations. The amount of N-acetylglucosamine and chito-oligosaccharides were measured with HPLC system (Violet, I) eluting with $H_2O$ as mobile phase and recording the eluate spectrophotometrically at 190 nm.

7. Enzyme assay: Samples were taken every 12-24 h and chitinolytic enzymes were assayed as follows: a mixture of 0.5 ml of 10 g/l colloidal chitin in citrate-phosphate buffer 0.05 M, pH 5.5 and 0.5 ml of the enzyme solution was incubated at 50°C for 10 min. The amount of reducing sugars released was measured by Miller's (1959) dinitrosalicylic acid method, using a standard curve for N-acetylglucosamine. One unit (U) of enzyme activity was taken as the amount of enzyme which released 1 $\mu$mol of N-acetylglucosamine per milliliter per minute under the assay conditions.

8. Scanning electron microscopy: For scanning electron microscopy, beads (Microcube BR-L01T(70)) were rinsed free of the growth medium with deionized water and, where needed, halved with a sharp lancet. After dehydration in an ethanol serie (50, 70, 80, 90% and absolute), the beads were critical-point dried and coated with gold. A Philips 505 (Philips, NL) scanning electron microscope was used.

Results relative to *P. janthinellum* P9 colonisation and chitinolytic enzymes production on different carriers are presented in table 3. It is shown that not all the carriers used appeared to allow the maximum level of fungal adhesion. The following carrier BR-LO1T(500), BRLO1T(70), FN-SO1T(500); FN-SO1B(70) and PUS seems to be particularly suitable for repeated batch cultivations presented hereafter.

Table 3

| Carrier | AEA (mU/ml) | MEA (mU/ml) | VP ($\mu$/l/h) | Colonisation (%) | Loose mycelum (g/l) |
|---|---|---|---|---|---|
| PUS | 151 ($\pm$15) | 176 (8) | 3.18 | < 100 | 1.5 |
| BR 70 | 295 ($\pm$32) | 342 (4) | 6.14 | 100 | 0.9 |
| BR 500 | 153 ($\pm$39) | 213 (8) | 3.13 | 100 | 0.8 |

Table 3 (continued)

| Carrier | AEA (mU/ml) | MEA (mU/ml) | VP (μl/h) | Colonisation (%) | Loose mycelum (g/l) |
|---|---|---|---|---|---|
| FN 70 | 250 (±43) | 318 (8) | 5.21 | < 100 | 2.9 |
| FN 500 | 189 (±28) | 250 (8) | 3.96 | < 100 | 3.4 |
| Legend: AEA= Average enzyme activity (9 repeated batches - 384 h); MEA= Maximum enzyme activity (in parenthesis the batch where MEA was achieved); VP= Volumetric productivity; Colonisation = Carrier colonisation. | | | | | |

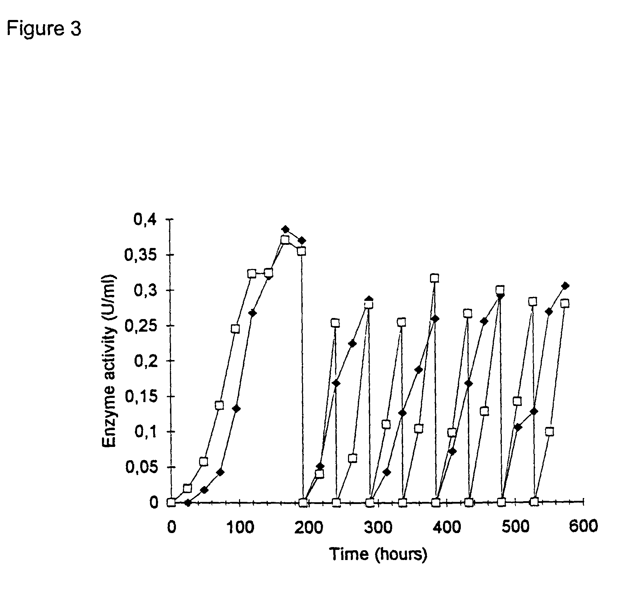

Repeated-batch cultivations were carried out as follows: after an activation time of 168 h, to induce the enzyme activity, the repeated batch process was started and 8 batches of 48 h each were carried out. Results show that, for the highest levels of enzyme production and volumetric productivity, the Macrocube BR-LO1T(70) appeared to be the most suitable, since it shows a complete beads colonisation and scarce release of loose mycelium. This carrier is thus particularly adapted for the continuous culture. Figure 3 shows the time course of repeated batch cultures of immobilised and free *P. janthinellum* cells. In both cases the levels of chitinolytic activity remained almost stable (ca. 0.3 U/ml) for all the batches (8 batches of 48 h and 4 of 96 h for immobilised and free cells, respectively). This result was somehow surprising and in contrast with what was reported for other microorganisms (Manolov, J. Microbiol. and Biotechnol., 9, 29-33, 1993; Petruccioli et al., Biotechnol. lett., 16, 939-942, 1994). In these cases, in fact, the activity of free cells decreased after the first batch, while the activity of the immobilised cells increased. In our case, however, the maximum enzyme activity of the immobilised mycelium was obtained in half the time necessary compared with the free mycelium; therefore, the amount of chitin processed (5.2 and 10.4 mg/h by the free and immobilised cells, respectively) and the volumetric productivity (2.98 and 5.89 U/l/h, respectively) were about double. The maximum level of enzyme activity (0.342 U/ml) was obtained with the immobilised cells on the 4th batch.

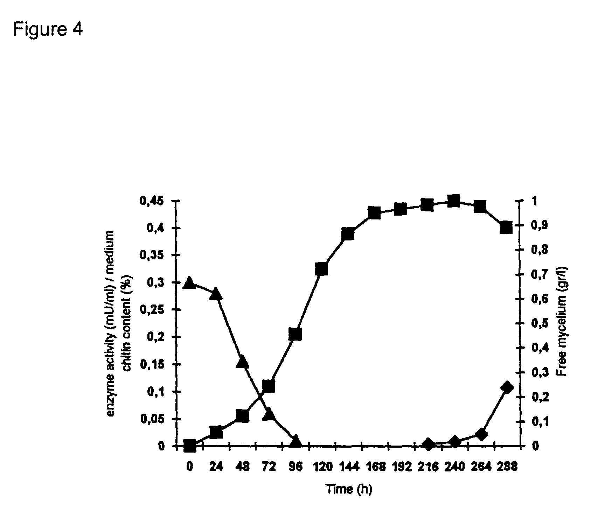

Continuous cultures were started after an activation batch of 120 h. The steady state appeared at the 144 h of cultivation and was maintained for over 120 h. Figure 4 shows the continuous production of chitinolytic enzymes by immobilised cells of *Penicillium janthinellum* P9. The maximum enzyme activity (0,450 U/ml) was obtained after 240 h of incubation. After that time, the enzyme activity started to decrease and a certain cell leakage from the carrier began.

Table 4 reports the comparison between semicontinuous cultures with free and immobilised cells and the continuous process. Maximum enzyme activity of the immobilised cells cultures in the bioreactor was 47.5% and 31.5% higher than that obtained in the repeated batch processes with free and immobilised cells, respectively. The volumetric productivity was 5 and 2 times higher, respectively. The enzyme yield, in the bioreactor, was 5 times higher than that obtained in the shaken cultures.

As shown for other enzymes and microorganisms, this study demonstrates the feasibility of using immobilised growing cells of *P. janthinellum* P9 for the repeated batch and continuous production of chitinolytic enzymes. In particular, the continuous process might be useful to degrade colloidal chitin in order to produce N-acetylglucosamine.

Table 4

| Fermentation | CV (ml) | N° | FT (h) | VB (ml) | CC (mg/h) | CHI (U/ml) | NAC (gr) | VP (U/l/h) | Yield (U/g) |
|---|---|---|---|---|---|---|---|---|---|
| Free cells Repeated batch | 50 | 4 | 384 | 200 | 5.2 | 0.0305 | 1.9 | 2.98 | 28.6 |
| Immob. cells Repeated batch | 50 | 8 | 384 | 400 | 10.4 | 0.342 | 3.5 | 5.89 | 28.3 |
| Immob cells Continuous | 1000 | ---- | 168 | 6048 | 108 | 0.450 | 15.9 | 14.76 | 137 |
| CV = Culture volume; N° = Number of batch carried out in the experiment; FT = Total fermentation time (excluded the activation time); VB = Total volume of the broth; CC = Amount of converted chitin; CHI = Maximum chitinolytic activity; NAC = Total amount of N-Acetyl glucosamine produced; VP = Volumetric productivity; Yield = chitinolytic enzyme units / g of chitin supplied. | | | | | | | | | |

Example 4 N-acetylglucosamine production

The aim was to obtain quantitative and qualitative data about the products resulting from the enzymatic hydrolysis of colloidal or raw chitin with a crude chitinolytic enzymes solution from *Penicillium janthinellum* P9. The final objective was to produce N-acetylglucosamine.

Chitinolytic enzymes from *P. janthinellum* were obtained using the continuous system described in example 3. The effluent was collected, filtered and precipitated in 80% ammonium sulphate overnight. The suspension was then centrifuged at 13.000 RPM for 10 min., and the pellet dissolved in 5 mM citrate-phosphate buffer pH 5.5, dialysed against the same buffer (4 changes in 24h) and lyophilised.

Colloidal chitin was prepared by treating chitin from crab shells (SIGMA) with 50% sulphuric acid and resuspending the dissolved material in distilled water (Hankin and Anagnostakis, 1975). After swelling, neutralisation by several washes and centrifugation, the colloidal chitin was sterilised by autoclaving 20 min at 121°C. The solid content of the sterile chitin suspension was evaluated by dry weight determination. Raw chitin was pre-treated using liquid nitrogen in a mortar with pestle, then by homogenisation (Polytron pt 3000) for 5 minutes at 16.000 RPM or by ultrasonic disintegration (B-Braun 2000U) for 5 minutes at 125 watts.

The substrate was hydrolysed by adding different solutions of enzyme activity (0.5, 1, 1.5, 2 UI/ml; the enzyme activity is determined according to example 3 analytical methods) to 0.5% native chitin or 0.82-0.89% colloidal chitin suspension made in 50 mM citrate-phosphate buffer at different pH. The reaction mixture was incubated for 48 h at different temperatures in a rotary shaker at 150 RPM.

The reducing sugars (RS), expressed as N-acetylglucosamine (GlcNAc) were determined by the Miller method. The production of N-acetylglucosamine and chitooligosaccharides $(GlcNAc)_n$ in the hydrolysed culture were qualitatively analysed by thin layer chromatography (TLC) and quantified by high performance liquid chromatography (HPLC). TLC was performed in a chamber saturated for 30 min with 10 ml solvent, and placed against one of the walls of the chamber. The solvent used was chloroform: glacial acetic acid: water (60:70:10). The amounts of sample and standard applied were 2 $\mu$l and 2 $\mu$l (5mg/ml of N acetylglucosamine, N-N' diacetylchitobiose, N-N'-N'' triacetylchitotriose in a mixture), respectively. Each sample or standard was applied slowly to the plate by a 10 $\mu$l-Hamilton syringe, and the use of a hairdryer. Three migrations were performed per plate. The solvent used was always the same but freshly replaced each time. The Whatman paper was also changed each time. The plate was dried well between each migration. After the third migration was accomplished, the plate was air-dried and the staining agent added. The staining agent was : 1 g diphenylamine, 1 ml aniline, 50 ml acetone and 7.5 ml *ortho*-phosphoric acid 85%. The plate was covered with the staining agent for 1 min, quickly air-dried and heated at 120°C for 30 min, in an oven.

The HPLC (Kontron instruments) conditions were as follows: column, Spherisorb $NH_2$ Aminopropyl, at room temperature; eluent acetonitrile: distilled water 75:25, at 2ml/min; RI-detector (ERC-7515A). The samples were filtered (membrane PTFE, pore size 0.2 $\mu$m) and diluted (1:2) in a mixture of acetonitrile: water (50:50). The concentration of each saccharide was estimated from the peak width on the chromatogram compared with that of the authentic $(GlcNAc)_n$.

The percentage of hydrolysis was determined as follows:

$$\% \text{ Hydrolysis} = \frac{\text{Amount of reducing sugars}}{\text{Amount of substrate}} \times \frac{203}{221} \times 100$$

Results show that it was possible to follow the formation of N-acetylglucosamine at the first hour of incubation without the detection of other chitooligosaccharides. This was in agreement with the high chitobiase activity present in the crude enzyme solution. The HPLC chromatograms from hydrolysis of raw chitin with 0.5 UI/ml enzyme present the same results.

Different experiments were carried out in order to find the best temperature and pH incubation for the N-acetylglucosamine production. The maximum yield of reducing sugars (83% and 12%) on hydrolysis was obtained at pH 5.5 after 72 hours of incubation, either for colloidal or raw chitin. The hydrolysis of colloidal chitin appeared to be better at 40°C (63.6% after 48 hours) whereas for raw chitin the maximum was reached at 35°C (9.4% after 48 hours). This cannot be explained by loss of enzyme stability since stability and activity of the crude enzyme solution is not affected.

Using colloidal chitin it was possible to obtain a production in the best optimum condition of 5.90 g/l of N-acetylglucosamine after 71 hours of incubation. Raw chitin was lacking in N-acetylglucosamine, giving only 0.44 g/l of the chitin monomer after 72 hours of hydrolysis. The utilisation of the substrate was 9% on raw chitin and 66.3% on colloidal chitin.

The rate of hydrolysis was faster during the first 12 hours followed by a decrease in the rate. While during the first 6 hours the quantity of N-acetylglucosamine produced represented about 41% for raw chitin and 66% for colloidal chitin of the total reducing sugars produced, during the time the N-acetylglucosamine and reducing sugars ratio increased up to maximum of 85% for raw chitin and 88% for colloidal chitin after 48 hours of incubation.

Example 5 N-acetylglucosamine production

In the same manner as that described in example 4, several chitins from different sources were used as hydrolytic substrates to test the different action of the chitinolytic complex of *P. janthinellum* P9 under optimal conditions. Chitins were prepared from crab shell, shrimp shell and fungal cell wall (*F. oxysporum* and *S. rolfii*).

There are distinct crystal structures in chitin. Two of the crystal structures are already well-known, such as the α-chitin structure, perhaps universally present in arthropod cuticles, and the less common β-chitin, which was recognised by Lotmar et al. (1950) and found in *Aphrodite* chaetae and in the pen of squid, *Loligo.* A third distinct form of chitin has not yet been characterised (Rudall, 1963). Results presented in table 5 below show that there was more enzyme hydrolytic activity on chitin from fungal source than on arthropod chitin. The different actions of the chitinolytic enzymes seems thus to depend on the different structures and association with proteins and calcium carbonate existing in the various chitin sources.

Table 5

| Substrate | Reducing sugars (mg/ml) | | | | | | | N-acetylglucosamine (mg/ml) | | | | | |
|---|---|---|---|---|---|---|---|---|---|---|---|---|---|
| | 12h | 24h | 36h | 48h | 60h | 70h | | 12h | 24h | 36h | 48h | 60h | 72h |
| Crab shells chitin | 0.32 | 0.40 | 0.46 | 0.51 | 0.51 | 0.57 | | 0.22 | 0.36 | 0.37 | 0.43 | 0.44 | 0.44 |
| Shrimp shells chitin | 0.24 | 0.26 | 0.32 | 0.33 | 0.33 | 0.37 | | 0.21 | 0.22 | 0.22 | 0.27 | 0.28 | 0.31 |
| Fungi cell wall chitin | | | | | | | | | | | | | |
| *F. oxysporum* | 0.34 | 0.44 | 0.59 | 0.73 | 0.95 | 1.15 | | 0.09 | 0.14 | 0.19 | 0.22 | 0.32 | 0.46 |
| *S. rolfii* | 0.49 | 0.49 | 0.49 | 0.52 | 0.53 | 0.53 | | 0.04 | 0.05 | 0.05 | 0.05 | 0.05 | 0.06 |

Example 6 Action of the crude chitinolytic solution of *P. janthinellum* P9 on food spoiling micro-organisms

Conidia from the following food spoiling strains were chosen for testing the ability of chitinolytic enzymes from *P. janthinellum* to inhibit food spoiling micro-organisms:

*Penicillium carneum, Penicillium granulatum, Penicillium citrinum, Penicillium corylophylum, Penicillium chrysogenum, Penicillium roquefortii, Penicillium hirsitum, Penicillium camembertii, Penicillium canescens, Penicillium brevicompactum, Fusarium monoliforme, Fusarium avenaceum, Fusarium equiseti, Fusarium solanii, Cladosporium cladosporoides, Cladosporium herbarum, Paecilomyces variotti, Eremicella nidulans, Mucor plumbeus, Byssochlamys nivea.*

All assays were performed under sterile conditions. Equal volume of spore suspension (500.000 conidia/ml) in YM medium and enzyme test (10UI/ml) solution were mixed and put on a sterile microscope slide. The enzyme solution was replaced with sterile distilled water in control samples. Slides were incubated overnight at 25°C in a sterile Petri dish. The percentage of conidia germinating was determined as the percentage of the first 100 spores randomly found on a microscope slide. In addition, the length of 100 germ tubes was measured with a calibrated ocular micrometer and averaged. Abnormal mycelial growth and morphological anomalies such as branching, bursting, appearance of necrotic zones, and lysis of the hyphal tips were also noted and photographed.

Results show that it is possible to find a certain inhibition in all the treated food spoiling strains. Difference in the degree of inhibition seems also to depend on the chitin percentage contained in the cell wall of several strains.

**Claims**

1. Chitinolytic enzymes produced by *P. janthinellum* cells, having a molecular mass by SDS-PAGE of about 40000-60000.

2. Chitinolytic enzymes extracted from *P. janthinellum* CNCM I-1869.

3. A process for producing chitinolytic enzymes, in which *P. janthinellum* cells are maintained in a medium under conditions suitable for the production of chitinolytic enzymes.

4. A process according to claim 3, wherein *P. janthinellum* cells are immobilised on a carrier.

5. A process according to claim 4, wherein the carrier is a chemically modified macroporous cellulose carrier.

6. A process according to claim 4, wherein chitinolytic enzymes are continuously produced.

7. A process according to any of claims 3 to 6, wherein *P. janthinellum* CNCM I-1869 is used.

8. A process according to any of claims 3 to 7, wherein chitinolytic enzymes are isolated from the medium.

9. A process for producing N-acetyl-glucosamine, in which the process according to claims 3-7 is carried out with a medium comprising chitin materials, and the N-acetylglucosamine thus produced is isolated.

10. Use of chitinolytic enzymes according to claims 1 or 2, for the production of chitin degradation products.

11. Use of chitinolytic enzymes according to claims 1 or 2, for inhibiting food spoiling micro-organisms.

12. Use of *Penicillium janthinellum* cells for the hydrolysis of chitin containing materials.

Figure 1

Figure 2

Figure 3

Figure 4

European Patent
Office

## EUROPEAN SEARCH REPORT

Application Number

EP 97 20 1833

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | JACKSON G.V.H. AND GAY J.L.: "Perennation of Sphaerotheca mors-uvae as cleistothecia with particular reference to microbial activity" TRANS. BR. MYCOL. SOC., vol. 66, no. 3, 1976, pages 463-471, XP002045891 | 2,3, 7-10,12 | C12N9/24 C12N1/14 C12N11/12 C12P19/26 C12P21/02 C12Q1/34 A23L3/3571 //(C12N1/14, C12R1:80) |
| Y | * abstract * * page 466, left-hand column, line 9-14 * | 4-6,11 | |
| Y | TANAKA H. ET AL.: "Efficient production of chitinase by Wasabia japonica protoplasts immobilized in double-layered fibers" JOURNAL OF FERMENTATION AND BIOENGINEERING, vol. 81, no. 5, 1996, pages 394-399, XP002045892 * abstract * | 4-6 | |
| Y | US 4 032 663 A (KOBAYASHI REISUKE ET AL) * column 15 - column 16; claims 5,6 * | 11 | TECHNICAL FIELDS SEARCHED (Int.Cl.6) |
| L | SHUNG-CHANG JONG, J.M. BIRMINGHAM, GUOZHONG MA: "ATCC names of industrial fungi" 1994 , AMERICAN TYPE CULTURE COLLECTION , US ROCKVILLE XP002045893 Synonyms of Penicillium janthinellum * page 194 * | | C12N C12P C12Q A23L |

-/--

The present search report has been drawn up for all claims

| Place of search | Date of completion of the search | Examiner |
|---|---|---|
| THE HAGUE | 13 November 1997 | Macchia, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

**EUROPEAN SEARCH REPORT**

Application Number

EP 97 20 1833

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | DATABASE WPI<br>Section Ch, Week 9206<br>Derwent Publications Ltd., London, GB;<br>Class D16, AN 92-044165<br>XP002045894<br>& JP 03 280 877 A (ASASHI SEIBUTSUKOGA),<br>11 December 1991<br>* abstract * | 3,8,10 | |
| A | WO 81 01714 A (PURDUE RESEARCH FOUNDATION)<br>25 June 1981<br>* abstract *<br>* page 14, line 7 - page 15, line 6 *<br>* page 16 - page 21; claims * | 4-6 | |

TECHNICAL FIELDS SEARCHED (Int.Cl.6)

The present search report has been drawn up for all claims

| Place of search | Date of completion of the search | Examiner |
|---|---|---|
| THE HAGUE | 13 November 1997 | Macchia, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document